(19) Europäisches Patentamt
European Patent Office
Office européen des brevets

(11) **EP 1 793 321 B1**

(12) **EUROPÄISCHE PATENTSCHRIFT**

(45) Veröffentlichungstag und Bekanntmachung des Hinweises auf die Patenterteilung:
**16.11.2016 Patentblatt 2016/46**

(51) Int Cl.:
***G06F 19/00*** *(2011.01)*

(21) Anmeldenummer: **05026421.7**

(22) Anmeldetag: **03.12.2005**

(54) **Auswerteverfahren und Untersuchungssystem eines Analyten im Körperflüssigkeit eines Menschen oder Tieres**

Evaluation method and analysis system of an analyte in the bodily fluid of a human or animal

Procédé d évaluation et système d analyse d'un analyte dans le fluide corporel humain ou animal

(84) Benannte Vertragsstaaten:
**AT BE BG CH CY CZ DE DK EE ES FI FR GB GR HU IE IS IT LI LT LU LV MC NL PL PT RO SE SI SK TR**

(43) Veröffentlichungstag der Anmeldung:
**06.06.2007 Patentblatt 2007/23**

(73) Patentinhaber:
• **Roche Diabetes Care GmbH
68305 Mannheim (DE)**
Benannte Vertragsstaaten:
**DE**
• **F.Hoffmann-La Roche AG
4070 Basel (CH)**
Benannte Vertragsstaaten:
**AT BE BG CH CY CZ DK EE ES FI FR GB GR HU IE IS IT LI LT LU LV MC NL PL PT RO SE SI SK TR**

(72) Erfinder:
• **Staib, Arnulf
64646 Heppenheim (DE)**
• **Kloetzer, Hans-Martin
68163 Mannheim (DE)**
• **Essenpreis, Matthias
3400 Burgdorf (CH)**

(74) Vertreter: **Mommer, Niels et al
Twelmeier Mommer & Partner
Westliche 56-58
75172 Pforzheim (DE)**

**Beschreibung**

Die Erfindung betrifft ein Verfahren zum Auswerten einer Serie von Meßdaten, die mit der Konzentration eines medizinisch bedeutsamen Analyten in einer menschlichen oder tierischen Körperflüssigkeit korrelieren. Die Erfindung betrifft ferner ein System zum Untersuchen des Stoffwechsels eines Menschen oder Tieres hinsichtlich eines medizinisch bedeutsamen Analyten. In diesem Zusammenhang wichtige Körperflüssigkeiten sind beispielsweise Blut und interstitielle Flüssigkeit sowie sonstige Flüssigkeiten, denen ein in Gewebe implantierter Sensor ausgesetzt sein kann.

Einer der wichtigsten medizinisch bedeutsamen Analyten in menschlichen Körperflüssigkeiten ist Glukose. Im folgenden wird deshalb ohne Beschränkung der Allgemeinheit auf Glukose als Beispiel für einen medizinisch bedeutsamen Analyten in einer menschlichen oder tierischen Körperflüssigkeit Bezug genommen.

Um an einer Serie von Messwerten der Glucosekonzentration krankheitsbedingte Besonderheiten erkennen zu können, wird in dem Artikel "Quantifying Temporal Glucose Variability in Diabetes via Continuous Glucose Monitoring: Mathematical Methods and Clinical Application" von B. Kovatchev et al. in Diabetes Technology & Therapeutics (2005); 7, 849-862, vorgeschlagen die Änderungsgeschwindigkeit der Blutglucosekonzentration zu untersuchen. Zur Risikobeurteilung und Charakterisierung der zeitlichen Dynamik werden dabei unter anderem das zeitliche Mittel und die Standardabweichung der ersten zeitlichen Ableitung von Absolutwerten der Glucosekonzentration vorgeschlagen.

Eine laufende Überwachung der Blutglucosekonzentration, bei der beispielsweise im Abstand von wenigen Minuten Meßwerte gewonnen werden, ist im Stand der Technik unter dem Schlagwort "continuous monitoring" beispielsweise aus der US 6272480 oder der EP 1102194 A2 bekannt. Bei diesen Anwendungen geht es darum, die zur Behandlung von Diabetes erforderlichen Insulingaben zu optimalen Zeitpunkten in optimalen Mengen zu verabreichen, um den Blutzuckerspiegel eines Diabetikers ähnlich wie bei einer gesunden Person ständig innerhalb enger Grenzen zu halten.

Die Blutglukosekonzentration eines Patienten ist von größter medizinischer Bedeutung. Studien haben zu dem Ergebnis geführt, daß äußerst schwerwiegende langfristige Folgen des Diabetes melitus (beispielsweise Erblinden aufgrund einer Retinopathie) vermieden werden können, wenn der Blutzuckerspiegel sorgfältig überwacht und innerhalb enger Grenzen gehalten wird.

Systeme zum Untersuchen und Überwachen des Glukosestoffwechsels verfügen über ein Sensormodul, das eine kontinuierliche oder quasi kontinuierliche Messung der Analytkonzentration ermöglicht. Geeignete Sensoren können beispielsweise direkt in Unterhautfettgewebe oder Blutgefäße implantiert werden. Ein solcher Sensor wird in Diabetes Technology & Therapeutics, Dezember 2000, 2(supplement 1) 13-18 beschrieben. Möglich ist es auch, Katheter zu implantieren, über die ein Austausch zwischen einem Dialysat und der umgebenden Körperflüssigkeit zum Sammeln von Analyten genutzt wird. Das Dialysat kann via Microfluidik zu einem außerhalb des Körpers angeordneten Sensor transportiert werden. Prinzipiell besteht ferner die Möglichkeit, Analytkonzentrationen mittels eines nichtinvasiven Sensors, der beispielsweise auf die Haut geklebt wird, zu messen. Eine Übersicht über Sensoren zur in vivo Messung der Glucoskonzentration gibt der Artikel "Sensors for glucose monitoring: technical and clinical aspects" von T. Koschinsky in Diabetes Metab Res Rev 2001; 17, 113-123.

Bekannte Systeme zum Überwachen der Glukosekonzentration verfolgen das Ziel, einem gefährlichen Anstieg der Blutglukosekonzentration durch eine Insulingabe zeitnah entgegen wirken zu können. Zu diesem Zweck wird häufig angestrebt, auf der Grundlage ermittelter Meßwerte künftige Blutglukosekonzentrationen über einen Zeitraum von etwa einer halben Stunde vorhersagen zu können, so daß durch eine rechtzeitige Insulingabe ein gefährlicher Anstieg der Glukosekonzentration verhindert werden kann (z. B. US 6272480). Ein Verfahren zur Vorhersage künftiger Glucosewerte ist auf der Grundlage einer Serie von Messwerten aus der WO 02/15777 A1 bekannt. Der Artikel "Hypothesis-Driven Data Abstraction with Trend Templates" von I. Kohane, Proc Annu Symp Comput Appl Med Care. 1993; 444-448. NLM8130513 beschreibt ein Computerprogramm zur automatischen Trenderkennung bei der Überwachung der Diabetestherapie.

Damit aus einem Roh- oder Meßsignal, beispielsweise einer Stromstärke, eines Sensors eine Analytkonzentration ermittelt werden kann, muß der verwendete Sensor aufwendig kalibriert werden. Grundvoraussetzung für eine erfolgreiche Kalibration ist, daß von dem Sensor ausgegebene Rohsignale mit Referenzwerten der Analytkonzentration die anhand von entnommenen Körperflüssigkeitsproben bestimmt werden, hinreichend korrelieren. Insbesondere bei implantierten Sensoren können sich Meßsensitivitäten im Lauf der Zeit gravierend ändern, so daß in regelmäßigen Zeitabständen eine erneute in-vivo Kalibration erforderlich sein kann. Probleme der Kalibration implantierbarer Sensoren und Ansätze zu deren Lösung sind in dem Artikel "Strategies for calibrating a subcutaneous glucose sensor" von G. Velho et al. in Biomed. Biochim. Acta 48 (1989) 957-964 zusammengefaßt.

Aus der DE 4221848 C2 ist ein Verfahren zur selbsttätigen in-situ Kalibrierung von implantierten Glucosesensoren bekannt, bei dem ein gleitender Mittelwert von Messwerten laufend mit einem durch ein mathematisches Modell ermittelten Referenzwert verglichen wird. Bei einer Abweichung von mehr als der doppelten Standardabweichung wird ein Alarmsignal ausgelöst und eine Rekalibration des Sensors oder ein Abbruch der Messung veranlasst.

Prinzipiell könnten Kalibrationsprobleme durch das Mitmessen eines internen Standards vermieden werden. Dieser Ansatz wird in dem Artikel "Electroosmosis in Transdermal Iontophoresis: Implications for Noninvasive and Calibration-Free Glucose Monitoring" von A. Sieg et al. Biophysical Journal, 87 (2004) 3344-3350 beschrieben.

Aufgabe der Erfindung ist es, einen Weg aufzuzeigen, wie krankheitsbedingte Besonderheiten des Stoffwechsels eines Menschen oder Tieres durch Auswerten einer Serie von Meßdaten mit reduziertem Kalibrationsaufwand, bevorzugt kalibrationsfrei, ermittelt werden können.

Diese Aufgabe wird gelöst durch ein System mit den im Anspruch 1 und ein Verfahren mit den im Anspruch 8 angegebenen Merkmalen.

Bevorzugt enthält die Serie Meßdaten in einer Dichte von mindestens drei Datenpunkten pro Stunde, besonders bevorzugt mindestens sechs Datenpunkten pro Stunde, insbesondere mindestens zehn Datenpunkten pro Stunde.

Bekannte Verfahren haben das Ziel, die Analytkonzentration möglichst präzise zu ermitteln und machen deshalb eine aufwendige Kalibration der verwendeten Sensoren erforderlich. Bei einem erfindungsgemäßen Verfahren kann jedoch darauf verzichtet werden, absolute Konzentrationswerte zu ermitteln, da sich krankheitsbedingte Besonderheiten in vielen Fällen bereits an der zeitlichen Dynamik, mit der sich Analytkonzentrationen in der Körperflüssigkeit ändern, erkennen lassen. Zur Untersuchung der zeitlichen Dynamik von Analytkonzentrationen genügt es, Meßdaten zu ermitteln, die mit der Analytkonzentration korrelieren, so daß absolute Konzentrationswerte nicht erforderlich sind und auf aufwendige Kalibrationen des verwendeten Sensors verzichtet werden kann.

Bevorzugt grenzen die ausgewählten Zeitintervalle aneinander an, möglich ist es jedoch auch, überlappende Zeitintervalle oder disjunkte Zeitintervalle zu wählen. Dabei kann mit der Auswertung einzelner Zeitintervalle bereits begonnen werden, bevor die Meßdatenserie vollständig vorliegt, also bevor der gesamte Zeitraum verstrichen ist, aus dem die Zeitintervalle ausgewählt werden.

Weitere Einzelheiten und Vorteile der Erfindung werden anhand eines Ausführungsbeispiels unter Bezugnahme auf die beigefügten Zeichnungen erläutert. Die darin dargestellten Besonderheiten können einzeln oder in Kombination verwendet werden um bevorzugte Ausgestaltung der Erfindung zu schaffen. Es zeigen:

Fig. 1  ein Beispiel von Rohdaten eines implantierten Sensors in Nanoampere über der Blutglukosekonzentration in mg/dl;

Fig. 2  ein Beispiel des Verlaufs von Meßwerten eines implantierten Glukosesensors über einen Zeitraum von 4 Tagen;

Fig. 3  mittels des erfindungsgemäßen Verfahrens ermittelte Stabilitätsparameter für verschiedene Probanden;

Fig. 4  eine vergleichende Darstellung der Stabilitätsparameter eines gesunden Probanden mit Stabilitätsparametern insulinpflichtiger Diabetiker für 5 aufeinanderfolgende Zeitintervalle; und

Fig. 5  eine schematische Darstellung eines erfindungsgemäßen Systems.

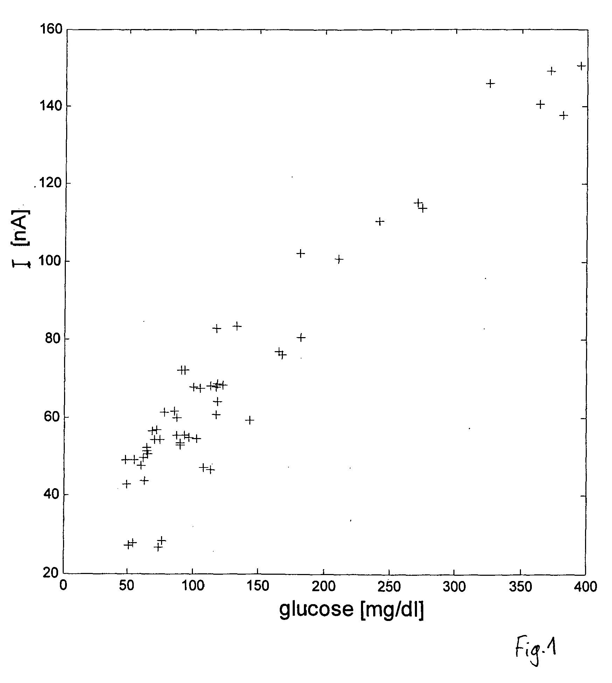

In Figur 1 sind Rohdaten in Nanoampere, die mit einem im Unterhautfettgewebe eines Probanden implantierten Sensor gemessen wurden, über der Blutglukosekonzentration in mg/dl aufgetragen. Der Glukosegehalt wurde mit einem konventionellen Blutzuckermeßgerät an Kapillarblut bestimmt.

Die in Figur 1 dargestellten Rohdaten könnten in Verbindung mit den zeitgleich gemessenen Konzentrationswerten der Abszisse zur Kalibration des Sensors verwendet werden. Im Rahmen der Erfindung ist dies jedoch nicht erforderlich. Bei dem im Folgendem beschriebenen Verfahren genügt es, daß mit der Blutglukosekonzentration korrelierende Meßwerte vorliegen.

Bei dem in Figur 1 dargestellten Beispiel zeigen die mit einem implantierten Sensor ermittelten Rohdaten abgesehen von Rausch- und Störsignalanteilen eine Proportionalität zu der Analytkonzentration. Nicht selten haben Sensoren jedoch nicht-lineare Funktionskurven, so daß die Rohdaten gemäß einer Funktionskurve nichtlinear transformiert werden, um Meßwerte zu erzeugen, die eine verbesserte Korrelation mit der Analytkonzentration zeigen, insbesondere der Analytkonzentration abgesehen von Rausch- und Störsignalen proportional sind.

Beispielsweise kann zwischen dem Meßstrom I eines Sensors und der Analytkonzentration c der folgende nicht lineare Zusammenhang bestehen:

$$I = I_0 + I_g(1-\exp(-c/c_r))$$

In dieser Funktionskurve ist $I_0$ ein Null- oder Untergrundstrom, der auftritt, wenn die Analytkonzentration c = 0 ist; $I_g$ ein Grenzstrom, der theoretisch bei einer unendlich großen Analytkonzentration c zu dem Null-Strom $I_0$ hinzukommt; und $c_r$ eine Referenzkonzentration, welche die Sensitivität des Sensor charakterisiert. Die Parameter $I_0$, $I_g$ und $c_r$ können ex-vivo bei der Herstellung für einen Sensortyp oder eine Produktionscharge mit geringem Aufwand bestimmt werden.

Bei Implantation eines solchen Sensors ändern sich insbesondere die Parameter Cr und $I_g$, so daß sich mit einer werkseitig bestimmten Funktionskurve keine absoluten Konzentrationswerte bestimmen lassen. Für das im folgenden beschriebene Verfahren ist dies jedoch auch nicht erforderlich. Es genügt, daß mittels einer derartigen Funktionskurve aus Rohdaten Meßwerte bestimmt werden können, die der Analytkonzentration abgesehen von Rausch- und Störsignalanteilen proportional sind, also eine hohe Korrelation mit der Analytkonzentration zeigen.

Je nach Art und Qualität der mit ermittelten Rohdaten können diese für das erfindungsgemäße Verfahren unmittelbar als Meßwerte verwendet werden oder die Meßwerte müssen zunächst aus Rohdaten berechnet werden, beispielsweise durch eine statistische Auswertung oder eine nicht-lineare Transformation gemäß der Funktionskurve des verwendeten Sensors.

Bei dem in Figur 1 dargestellten Beispiel beträgt der Korrelationskoeffizient zwischen den Rohdaten und der Glucosekonzentration R=0,95, so daß diese unmittelbar als Meßwerte verwendet werden können. Es empfiehlt sich aus stark verrauschten Rohdaten, die nur eine relative geringe Korrelation mit der Glukosekonzentration zeigen, durch statistische Auswertung oder geeignete Filteralgorithmen Meßwerte zu erzeugen, die eine gegenüber den zugrunde liegenden Rohdaten deutlich verbesserte Korrelation mit der Analytkonzentration zeigen.

Im Rahmen der Anmeldung ist dabei unter dem Begriff Korrelation auch eine Antikorrelation zu verstehen, da eine Multiplikation der Meßwerte mit einem Faktor von -1 an den wesentlichen Zusammenhängen zwischen den Meßwerten und den zugrundeliegenden Analytkonzentrationen nichts ändert. Bevorzugt werden mit dem im folgendem beschriebenen Verfahren Meßwerte ausgewertet, deren Korrelationskoeffizient mit der Glukosekonzentration einen Betrag von mindestens 0,5, bevorzugt mindestens 0,7, besonders bevorzugt mindestens 0,9 hat. Prinzipiell ist das Verfahren jedoch auch auf Meßdaten mit schlechteren Korrelationskoeffizienten anwendbar, wobei jedoch die Aussagekraft der erhaltenen Ergebnisse in derartigen Fällen entsprechend geringer ist.

Wichtig für das Verständnis des im folgenden beschriebenen Verfahrens ist, daß sich an dem in Figur 1 dargestellten Korrelationsbild und insbesondere dem Betrag des Korrelationskoeffizienten durch Anwendung einer linearen Transformation f nichts ändert. Eine lineare Transformation läßt sich allgemein in der Form f=ax+b schreiben. Dies bedeutet, daß bei einer linearen Transformation ein Meßwert x mit einem konstanten Faktor a multipliziert und zu dem Ergebnis eine Konstante b addiert wird. Geometrisch entspricht dies bei dem in Figur 1 dargestellten Beispiel einer Streckung oder Stauchung der Ordinatenachse und einer Verschiebung der Meßwerte in Richtung der Ordinatenachse.

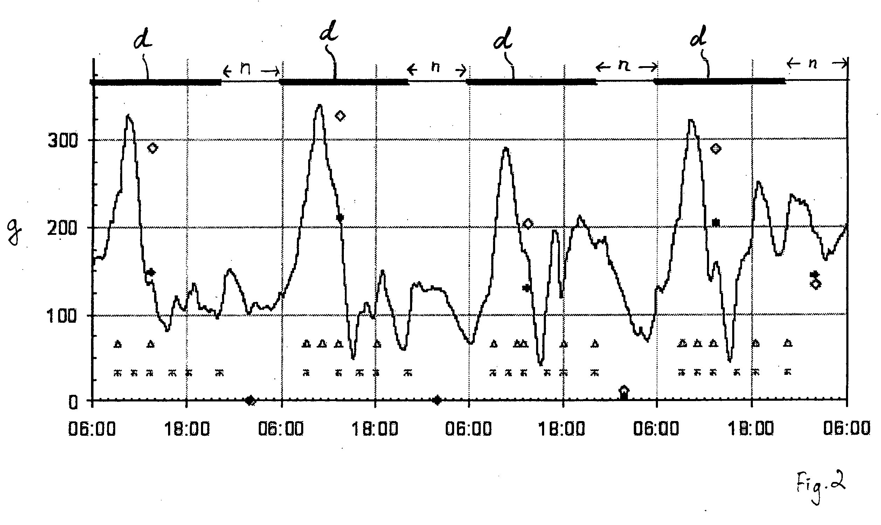

In Figur 2 ist eine Serie von quasi kontinuierlichen Meßdaten g in willkürlichen Einheiten über der Zeit t für ein Zeitraum von 4 Tagen aufgetragen. Dabei sind auf der Abszisse zu den Meßdaten gehörende Uhrzeiten aufgetragen. Den in Figur 2 dargestellten Meßdaten liegen linear transformierte Meßwerte wie sie in Figur 1 dargestellt sind, zugrunde, die retrospektiv mit einem Medianfilter und einem adaptiven rekursiven Filter geglättet wurden.

Aus dem in Figur 2 dargestellten Zeitraum von 4 Tagen wurden Zeitintervalle d, n ausgewählt, die bei dem dargestellten Ausführungsbeispiel Tag- und Nachtzeiten entsprechen und folglich den Ablauf der Analytkonzentration für Wachzeiten und nächtliche Ruhezeiten widerspiegeln. Allgemein ist es günstig aus dem Zeitraum, über den die Zeitpunkte $t_1$ bis $t_n$, für welche die Meßdaten $g(t_1)$ bis $g(t_n)$ der Serie gelten, verteilt sind, Zeitintervalle auszuwählen, die mit charakteristischen Phasen des untersuchten Stoffwechsels korrelieren, wie dies beispielsweise bei prä- und postprandialen Phasen oder Tag- und Nachtzeiten der Fall ist.

Für die ausgewählten Zeitintervalle wird jeweils durch Auswertung von in dem betreffenden Zeitintervall liegenden Meßdaten g ein Stabilitätsparameter ermittelt, der die zeitliche Dynamik, mit der sich die Konzentration des Analyten in dem Zeitintervall ändert, charakterisiert. Dieser Stabilitätsparameter wird ausgewertet, um krankheitsbedingte Besonderheiten des Stoffwechsels zu ermitteln. Auf diese Weise kann eine beginnende Diabeteserkrankung erkannt werden oder bei einem insulinpflichtigen Diabetiker im Fall einer ermittelten krankheitsbedingten Besonderheit des Glukosestoffwechsels eine Empfehlung zur Einstellung von Insulingaben zugeordnet werden.

Zur Berechnung des Stabilitätsparameters werden zunächst aus Meßwerten, wie sie in Figur 1 dargestellt sind, Meßdaten g berechnet, wobei als ein Berechnungsschritt eine lineare Transformation f vorgenommen wird. Bevorzugt werden zusätzlich weitere Berechnungsschritte vorgenommen, bei denen die Meßwerte vor oder nach dem Durchführen der linearen Transformation f mit geeigneten Filteralgorithmen und statistischen Methoden aufbereitet und geglättet werden.

Zur Berechnung des Stabilitätsparameters werden zunächst aus Meßwerten, wie sie in Figur 1 dargestellt sind, Meßdaten g berechnet, wobei als ein Berechnungsschritt eine lineare Transformation f vorgenommen wird. Bevorzugt werden zusätzlich weitere Berechnungsschritte vorgenommen, bei denen die Meßwerte vor oder nach dem Durchführen der linearen Transformation f mit geeigneten Filteralgorithmen und statistischen Methoden aufbereitet und geglättet werden.

Sofern die Meßsensitivität des verwendeten Sensors zeitlich hinreichend konstant ist, kann für mehrere Intervalle dieselbe Transformation verwendet werden. Häufig ändert sich bei implantierten Sensoren jedoch die Meßsensitivität und/oder das Untergrundsignal, so daß vorzugsweise für unterschiedliche Intervalle individuell eine Transformation f festgelegt wird.

Die lineare Transformation f wird dabei für die einzelnen Zeitintervalle jeweils derart gewählt, daß der Mittelwert der Meßdaten g des betreffenden Zeitintervalls einem vorgegebenen Wert entspricht. Bevorzugt ist dieser vorgegebener Wert 0, prinzipiell kann jedoch auch eine beliebige andere Konstante gewählt werden. Beispielsweise kann die lineare Transformation f auch so gewählt werden, daß Intervallgrenzen vorgegeben werden und der kleinste Meßdatenpunkt der unteren Intervallgrenze, beispielsweise dem Wert 0, und der größte Meßdatenpunkt der oberen Intervallgrenze, beispielsweise dem Wert 1, zugeordnet wird.

Da eine lineare Transformation f gemäß der Gleichung f=ax+b zwei wählbare Parameter, nämlich die Steigung a und eine additive Konstante b enthält, sind die linearen Transformationen f durch die Vorgabe eines Mittelwerts bestenfalls quasi kontinuierlich, vorliegen, wird die erste zeitliche Ableitung g' numerisch gebildet, beispielsweise mit einem Polynomfilter. In einem weiteren Berechnungsschritt wird die Standardabweichung der Werte der zeitlichen Ableitung g' des betreffenden Intervalls berechnet.

Die so ermittelte Standardabweichung charakterisiert die zeitliche Dynamik, mit der sich die Konzentration des Analyten in dem betrachteten Zeitintervall ändert.

Der Glukosestoffwechsel eines gesunden Probanden zeichnet sich dadurch aus, daß körpereigene Regulationsmechanismen einem durch Nahrungsaufnahme bedingten Anstieg der Glukosekonzentration rasch entgegenwirken, so daß die Standardabweichung der Werte der zeitlichen Ableitung g' verhältnismäßig groß ist. Auf einen raschen Anstieg folgt eine rasche Abnahme, so daß in einem Zeitintervall sowohl hohe positive als auch hohe negative Werte der ersten zeitlichen Ableitung g' auftreten.

Bei einem Diabetiker sind die körpereigenen Regelungsmechanismen gestört, so daß erhöhte Werte der Glukosekonzentration nur recht langsam abgebaut werden. Typischerweise treten bei einem Diabetiker deshalb hohe positive und kleine negative Werte der ersten zeitlichen Ableitung g' auf. Eine Diabeteserkrankung führt deshalb dazu, daß die Standardabweichung der Werte der zeitlichen Ableitung g' deutlich kleiner als bei einer gesunden Vergleichsperson ist.

Durch Auswertung des Stabilitätsparameters, beispielsweise durch Zuordnung zu vorgegebenen Parameterbereichen, kann eine krankheitsbedingte Besonderheit des Stoffwechsels ermittelt werden, insbesondere eine Diabeteserkrankung bzw. das Stadium einer Diabeteserkrankung diagnostiziert werden. Durch Auswertung der Stabilitätsparameter kann den ermittelten krankheitsbedingten Besonderheiten des Glukosestoffwechsels auch eine Empfehlung zur Einstellung der Dosierung von Insulingaben eines insulinpflichtigen Diabetikers zugeordnet werden.

Die optimale Dosierung von Insulingaben bereitet nach dem Stand der Technik erhebliche Probleme. In der Praxis beruhen gewählte Insulindosierungen zu einem erheblichen Teil auf Erfahrungswerten des behandelnden Arztes oder auch des Patienten selbst. Typischerweise wird für einen Diabetiker von einem Arzt ein Dosierungsplan erstellt, der einerseits Menge und Häufigkeit von Insulingaben zur Deckung eines Insulingrundbedarfs vorgibt und ferner Instruktionen enthält, wie als Reaktion auf erhöhte Meßwerte der Glukosekonzentration und Mahlzeiten zusätzliche Insulingaben dosiert werden sollen. Insulingaben zur Deckung des Insulingrundbedarfs werden in diesem Zusammenhang als Basalrate und zusätzliche Insulingaben im Zusammenhang mit Mahlzeiten als Bolus bezeichnet. Die allgemeine Dosierungsanweisung, nach der ein Diabetiker die Dosierung der zu verabreichenden Insulingaben ermittelt, bezeichnet man als Einstellung.

Neben der Einstellung von insulinpflichtigen Diabetikern umfaßt das sogenannte Diabetes-Management eine Reihe weiterer wesentlicher Punkte, um die Wahrscheinlichkeit von Stoffwechselentgleisungen zu reduzieren (E. Standl et al: Grundlagen des Diabetes-Managements, in Diabethologie in Klinik und Praxis, Herausgeber H. Mehnert et al., Thieme Verlag, Stuttgart, 2003, Seite 132 ff). Wichtigste Komponente des Diabetes-Managements ist neben der Einstellung die Stoffwechselselbstkontrolle, primär des Glukosespiegels, unter Umständen aber auch von kumulativen Parametern wie Ketonkörperkonzentrationen, HbA1c, oder glykolisierten Serumproteinen. Zum Diabetes-Management gehören typischerweise auch nicht medikamentöse Therapiemaßnahmen (z.B. Ernährungsplan, körperliche Aktivität) und insbesondere bei Typ 2 Diabetikern, medikamentöse Maßnahmen wie orale Antidiabetika. Eine wichtige Komponente des Diabetes-Managements stellt ferner das Monitoring des Gesamtrisikoprofils dar, speziell hinsichtlich diabetesbezogener Spätschäden, wobei ergänzend Untersuchungen der Nierenfunktion, des Lipidprofils und des Blutdrucks hinzugezogen werden können. Ein zentraler Bestandteil eines Diabetes-Managementsystems ist dabei die Langzeitanwendung eines Dokumentationssystems, in dem die vorgenannten Daten zur Stoffwechselselbstkontrolle und zur Einstellung, aber auch zur Ernährung und anderen relevanten Ereignissen gespeichert werden. Das beschriebene Verfahren kann einen wichtigen Beitrag zu einem Diabetes-Managementsystem liefern, da durch Auswertung der Stabilitätsparameter wichtige Daten über krankheitsbedingte Besonderheiten des Stoffwechsels ermittelt werden können.

Mit dem beschriebenen Verfahren lassen sich auch ohne Kenntnis absoluter Glukosekonzentrationswerte durch Auswertung der Stabilitätsparameter Empfehlungen zur Einstellung der Dosierung von Insulingaben oder allgemein zum Diabetes-Management, beispielsweise zu nicht-medikamentösen Therapiemaßnahmen, ermitteln. Tritt beispielsweise in einem Zeitintervall nach einer Mahlzeit ein starker Anstieg der Glukosekonzentration auf, der nur langsam oder unvollständig abgebaut wird, ist die Standardabweichung der Werte der zeitlichen Ableitung g' der Meßdaten g kleiner als dies bei einer raschen und vollständigen Rückkehr der Glukosekonzentrationen in die physiologische Gleichgewichtskonzentration der Fall wäre. In einem solchen Fall wäre es angezeigt, den Bolus der Insulingaben zu erhöhen. Alternativ kann im Rahmen des Diabetes-Managements beispielsweise auch die Empfehlung gegeben werden, die Aufnahme von Broteinheiten bei einer Mahlzeit zu reduzieren oder nach einer Mahlzeit durch körperliche Betätigung dem Anstieg der Glukosekonzentration entgegenzuwirken. Durch Auswertung der Stabilitätsparameter von Zeitintervallen, in denen keine Mahlzeiten eingenommen werden, kann überprüft werden, ob die eingestellte Basalrate den Bedürfnissen des Patienten entspricht.

Um die beschriebene Auswertung der Meßdaten g vornehmen zu können und diese aus Meßwerten gemäß Figur 1 zu erzeugen, empfiehlt sich der Einsatz eines Computers. Das beschriebene Verfahren wird deshalb bevorzugt als Computerprogrammprodukt realisiert, das direkt in den Speicher eines digitalen Computers geladen werden kann und Softwareabschnitte umfaßt, mit denen die Schritte des beschriebenen Verfahrens ausgeführt werden, wenn das Produkt auf einem Computer läuft.

Um krankheitsbedingte Besonderheiten des untersuchten Stoffwechsels mit möglichst großer Zuverlässigkeit ermitteln zu können, werden mehrere Stabilitätsparameter ausgewertet. Bevorzugt wird dabei aus den Stabilitätsparametern verschiedener Zeitintervalle ein Stabilitätsvektor ermittelt, dessen Komponenten jeweils für ein Zeitintervall die zeitliche Dynamik, mit der sich die Konzentration des Analyten in dem betreffenden Zeitintervall ändert, charakterisieren. Im einfachsten Fall sind die Komponenten des Stabilitätsvektors die für die betreffenden Intervalle ermittelten Stabilitätsparameter.

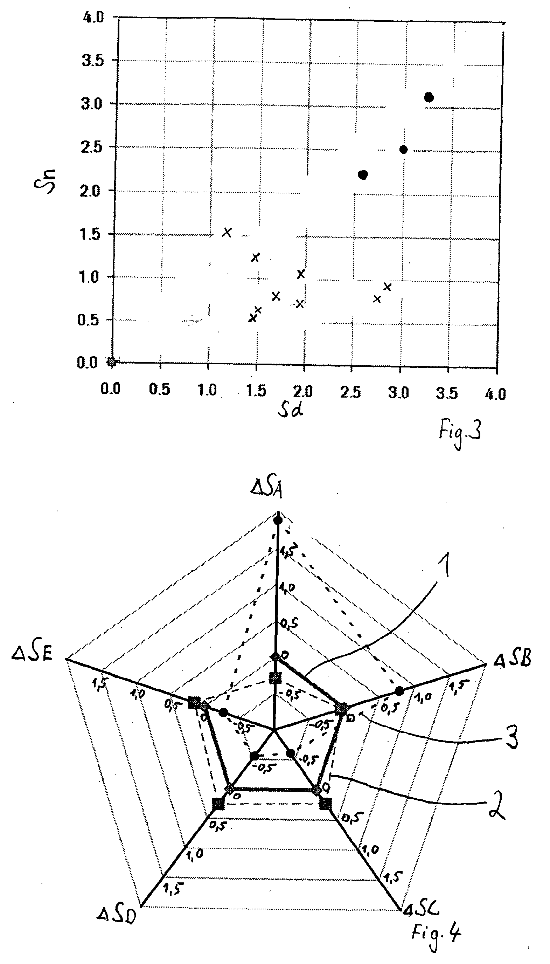

In Figur 3 sind Beispiele eines derartigen Stabilitätsvektors für verschiedene Probanden aufgetragen. Der in Figur 3 dargestellte Stabilitätsvektor hat zwei Komponenten, nämlich einen Stabilitätsparameter Sd für Wachzeiten der Probanden (06.00 Uhr bis 22.00 Uhr) und einen Stabilitätsparameter Sn für nächtliche Ruhezeiten (22.00 Uhr bis 06.00 Uhr). Die entsprechenden Zeitintervalle d, n sind in Figur 2 angedeutet. In Figur 3 gibt die Abszisse den Wert des Stabilitätsparameters Sd für Wachzeiten und die Ordinate den Wert des Stabilitätsparameters Sn für nächtliche Ruhezeiten in willkürlichen Einheiten an. Stabilitätsvektoren gesunder Probanden sind in Figur 3 durch Kreise (●), Stabilitätsvektoren von Diabetikern durch Kreuze (X) dargestellt.

Man erkennt in Figur 3, daß die Werte der Stabilitätsparameter von Diabetikern insbesondere bei Nacht deutlich geringer als bei gesunden Probanden sind. Dies liegt unter anderem daran, daß die (geschädigten) körpereigenen Regelungsmechanismen insulinpflichtiger Diabetiker tagsüber durch externe Insulingaben unterstützt werden. Bei optimaler Einstellung der Insulingaben können deshalb für den Stabilitätsparameter Sd für Wachzeiten Werte erreicht werden, die mit Werten gesunder Probanden vergleichbar sind. Nachts fehlt es jedoch an einer vergleichbaren Unterstützung der körpereigenen Regelungsmechanismen durch externe Insulingaben, so daß sich krankheitsbedingt eine schlechte Kontrollstabilität der Konzentrationswerte ergibt und folglich kleinere Werte des Stabilitätsparameters Sn beobachtet werden.

eine schlechte Kontrollstabilität der Konzentrationswerte ergibt und folglich kleinere Werte des Stabilitätsparameters Sn beobachtet werden.

Ein erfindungsgemäßer Stabilitätsparameter für eine derartige Anwendung wird mittels einer Frequenzanalyse der ersten oder der zweiten zeitlichen Ableitung g' bzw. g'' der Meßdaten g erhalten. Für ein hinreichend langes zeitliches Fenster sind die zeitlichen Ableitungen praktisch stationär, d.h. sie weisen über diesem Fenster keinen signifikanten positiven oder negativen Trend auf. Eine gute Kontrollstabilität im Stoffwechsel zeigt sich dann in einer Häufung von Fluktuationen im zeitlichen Verlauf von g' oder g''. Eine Fourier-Transformation der zeitlichen Ableitung g' oder g'', speziell die Berechnung eines Powerspektrums, ermöglicht die Analyse dieser Fluktuationen.

Eine schlechte Kontrollstabilität führt dazu, daß in dem Powerspektrum vermehrt niedrige Frequenzen auftreten. Als Stabilitätsparameter kann deshalb beispielsweise das Verhältnis der spektralen Intensität hoher Frequenzen zu der spektralen Intensität niedriger Frequenzen in dem Powerspektrum der zeitlichen Ableitung g' der Meßdaten g sein. In entsprechender Weise kann als Stabilitätsparameter auch das Verhältnis der spektralen Intensität hoher Frequenzen im Verhältnis zu der spektralen Intensität niedriger Frequenzen des Powerspektrums der zweiten zeitlichen Ableitung g'' der Meßdaten g verwendet werden.

Prinzipiell kann durch Einsatz programmierbarer Insulinpumpen auch in den Nachtphasen die Kontrollstabilität der Glukosekonzentration verbessert werden. Durch Auswertung der mit dem beschriebenen Verfahren ermittelten Stabilitätsparameter kann dabei die Pumprate einer derartigen die Pumprate für die betreffende Tageszeit erhöht, beziehungsweise gesenkt, wird.

In diesem Zusammenhang ist zu beachten, daß die meisten Menschen einen regelmäßigen Tagesablauf haben und folglich auch die zeitlichen Dynamik, mit der sich die Konzentration des Analyten ändert, von einem 24-Stunden Rhythmus geprägt ist. Erkenntnisse, die beispielsweise für nächtliche Ruhezeiten durch Beobachtung im Lauf einiger Tage gewonnen wurden, sind deshalb auf künftige nächtliche Ruheperioden übertragbar. Aus diesem Grund können zur Verbesserung der Zuverlässigkeit der erzielten Ergebnisse Stabilitätsparameter vergleichbarer Zeitintervalle statistisch ausgewertet werden, beispielsweise durch Mittelwertbildung.

Im einfachsten Fall sind Zeitintervalle, die durch identische Uhrzeiten begrenzt sind, stets vergleichbar. Der Beginn eines Intervalls kann jedoch auch durch ein relevantes Tagesereignis, insbesondere die Einnahme einer Mahlzeit, festgelegt werden. Diese Vorgehensweise empfiehlt sich insbesondere bei Personen mit einem eher unregelmäßigen Tagesablauf.

Bei dem in Figur 4 dargestellten Beispiel wurden die Nachtphasen n der in Figur 2 dargestellten Meßdaten g jeweils in fünf aufeinanderfolgende Intervalle A, B, C, D, E von jeweils 1,6 Stunden Dauer unterteilt und die für die einzelnen Intervalle der verschiedenen Nächte ermittelten Stabilitätsparameter jeweils für das betreffende Intervall gemittelt. Auf diese Weise wurde ein Stabilitätsvektor gebildet, der fünf Komponenten SA, SB, SC, SD, SE aufweist, wobei jeder der fünf Komponenten ein Mittelwert aus vier Stabilitätsparametern ist, die für das betreffende Intervall in den vier Nächten der Figur 2 ermittelt wurden.

In Figur 4 ist für drei Probanden die Abweichung $\Delta$SA, $\Delta$SB, $\Delta$SC, $\Delta$SD, $\Delta$SE der Komponenten SA, SB, SC, SD und SE des so ermittelte Stabilitätsvektors von einem Idealwert (2,5 in den willkürlichen Einheiten von Figur 3) in einem Pentagramm angegeben. Von dem Mittelpunkt des Pentagramms verläuft durch dessen Ecken jeweils eine Achse, die den Wert der Abweichung $\Delta$S des Stabilitätsparameter S von dem Idealwert in dem betreffenden Zeitintervall angibt.

Selbstverständlich kann diese Methode auch auf einen ganzen Tag oder einen anderen Zeitabschnitt angewendet oder/und eine andere Unterteilung vorgenommen werden. In einem solchen Fall ergibt sich bei einer Darstellung gemäß Figur 4 ein N-Eck-Diagramm, wobei jeder Ecke des Diagramms eine Komponente eines N-komponentigen Stabilitätsvektors zugeordnet ist. Bei einem gesunden Probanden ist die Abweichung $\Delta$S des Stabilitätsparameters S von dem Idealwert definitionsgemäß Null.

In Figur 4 gibt die Linie 1 als Referenz den idealen Verlauf der Abweichungen $\Delta$S=0 der Stabilitätsparameter eines gesunden Probanden an. Zum Vergleich sind die Ergebnisse von zwei Diabetikern durch gestrichelte Linien 2, 3 dargestellt. Vergleicht man den Verlauf der Linien 2, 3 der insulinpflichtigen Diabetiker mit der Linie 1 des gesunden Probanden, fällt auf, daß die Linie 2 relativ geringe Abweichungen von dem idealen Verlauf des gesunden Probanden zeigt, was darauf schließen läßt, daß bei dem betreffenden Probanden nur geringe krankheitsbedingte Besonderheiten des Stoffwechsels vorhanden sind. Die Linie 3, welche die Abweichung $\Delta$S der Stabilitätsparameter für den anderen Diabetiker angibt, zeigt jedoch eine deutliche Abweichung von dem idealen Verlauf. Dies zeigt, daß bei dem betreffenden Patienten die Einstellung der Insulingaben angepaßt werden sollte.

Bei einer Darstellung gemäß Figur 4 läßt sich durch eine Auswertung der Flächen zwischen den Linien 2, 3 eines zu untersuchenden Probanden und der Referenzlinie die Güte der glykämischen Selbstkontrolle und damit auch die Güte der Einstellung von Insulindosierungen ermitteln.

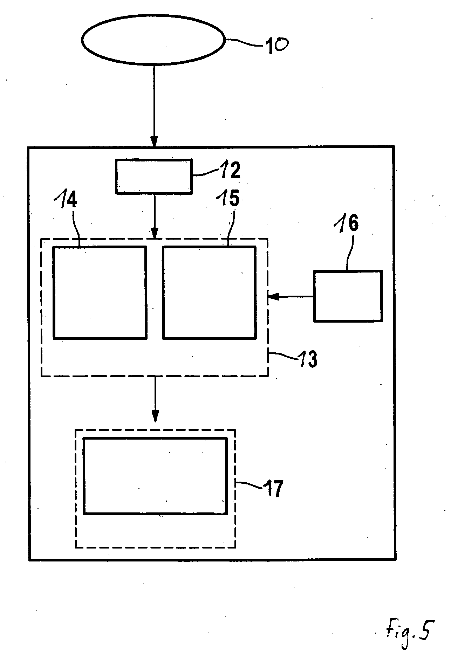

In Figur 5 sind die wesentlichen Komponenten eines Systems dargestellt, mit dem sich der Stoffwechsel eines Menschen oder Tieres gemäß dem im vorhergehenden beschriebenen Verfahren untersuchen läßt. Eine Meßeinheit mißt mit einem Sensor 10 zu aufeinanderfolgenden Zeitpunkten $t_n$ Meßwerte. Die Meßwerte werden - im dargestellten Fall drahtlos - an einen Empfänger 12 übertragen, von dem die Meßwerte an eine Auswerteeinrichtung 13 weitergeleitet werden, die einen Mikroprozessor 14 und einen Datenspeicher 15 enthält. Die Ausgabe von Resultaten erfolgt mittels einer Ausgabeeinheit 17, die ein Display oder andere übliche Ausgabemittel einschließen kann. Selbstverständlich erfolgt die Datenverarbeitung der Auswerteeinrichtung 13 digital und es sind entsprechende Wandler zur Umwandlung analoger Signale in digitale Signale vorgesehen.

Das System umfaßt ferner eine Eingabeeinheit 16, über die Daten oder Befehle an die Auswerteeinrichtung 13 übermittelt werden können. Indem beispielsweise zu Beginn einer Nachtphase an einer entnommenen Körperflüssigkeitsprobe mittels eins handelsüblichen Teststreifens und eines dazugehörenden Testgeräts ein Blutzuckerwert ermittelt und der Auswerteeinrichtung 13 zur Verfügung gestellt wird, kann von der Auswerteeinrichtung der Verlauf der Glukosekonzentration während der Nachtphase geschätzt werden, insbesondere um anzugeben, ob eine gefährliche Unterschreitung oder Überschreitung des normoglykämischen Bereiches geschieht.

In dem Datenspeicher 15 werden die Stabilitätsparameter, die mit dem im vorhergehenden beschriebenen Verfahren ermittelt wurden, gespeichert, um für eine Langzeitauswertung im Rahmen des Diabetes-Managements zur Verfügung zu stehen. Mittels der Ausgabeeinheit 17 werden dem Benutzer des Systems die von den Stabilitätsparametern abgeleiteten therapeutischen Empfehlungen ausgegeben. Bevorzugt werden diese Empfehlungen ebenfalls in dem Speicher 15 gespeichert. Auf diese Weise kann mit dem System der Erfolg von therapeutischen Empfehlungen bewertet werden, beispielsweise in dem eine Stabilitätsanalyse von Sensordaten durchgeführt wird, die in einem bestimmten Zeitraum nach einer empfohlenen therapeutischen Handlung aufgenommen wurden.

**Patentansprüche**

1. System zum Untersuchen des Stoffwechsels eines Menschen oder Tieres hinsichtlich eines medizinisch bedeutsamen Analyten, umfassend
einen Sensor (10) zum Ermitteln von Messwerten der Konzentration eines medizinisch bedeutsamen Analyten in einer menschlichen oder tierischen Körperflüssigkeit, und
eine Auswerteeinrichtung (13) zum Auswerten einer Serie von mittels des Sensors (10) ermittelten Messwerten,
wobei die Auswerteeinrichtung (13) derart eingerichtet ist, dass sie im Betrieb aus einem Zeitraum von mindestens 8 Stunden, über den die Messwerte der Serie verteilt sind, mehrere Zeitintervalle auswählt, die sich jeweils über mindestens 1 Stunde erstrecken, und aus den Messwerten Messdaten g berechnet, wobei als Berechnungsschritt eine lineare Transformation vorgenommen wird, die für unterschiedliche Intervalle individuell festgelegt wird,
die erste zeitliche Ableitung g' von Messdaten g bildet,
**dadurch gekennzeichnet, dass** die Auswerteeinrichtung (13)für die Zeitintervalle jeweils aus der ersten zeitlichen Ableitung g' oder der zweiten zeitlichen Ableitung g'' der in dem betreffenden Zeitintervall liegenden Messdaten g durch eine Fourier-Transformation ein Powerspektrum berechnet und aus dem Verhältnis der spektralen Intensität hoher Frequenzen zu der spektralen Intensität niedriger Frequenzen einen Stabilitätsparameter S berechnet, der die zeitliche Dynamik, mit der sich die Konzentration des Analyten in dem Zeitintervall ändert, charakterisiert, und
die Stabilitätsparameter S auswertet, um krankheitsbedingte Besonderheiten des Stoffwechsels zu ermitteln.

2. System nach Anspruch 1, **dadurch gekennzeichnet, dass** von der Auswerteeinrichtung (13) in einem weiteren Berechnungsschritt zur Bestimmung des Stabilitätsparameters die Standardabweichung der Werte der zeitlichen Ableitung g' berechnet wird.

3. System nach einem der vorstehenden Ansprüche, **dadurch gekennzeichnet, dass** von der Auswerteeinrichtung (13)die lineare Transformation f für die einzelnen Zeitintervalle jeweils derart gewählt wird, dass der Mittelwert der Messdaten g des betreffenden Zeitintervalls einem vorgegebenen Wert entspricht.

4. System nach einem der vorstehenden Ansprüche, **dadurch gekennzeichnet, dass** von der Auswerteeinrichtung (13) die lineare Transformation f für die einzelnen Zeitintervalle jeweils derart gewählt wird, dass die Standardabweichung der Messdaten g des betreffenden Zeitintervalls einem vorgegebenen Wert entspricht.

5. System nach einem der vorhergehenden Ansprüche, **dadurch gekennzeichnet, dass** der Analyt Glukose ist.

6. System nach Anspruch 5, **dadurch gekennzeichnet, dass** von der Auswerteeinrichtung (13) den durch Auswertung der Stabilitätsparameter ermittelten krankheitsbedingten Besonderheiten des Glukosestoffwechsels Therapieempfehlungen, insbesondere zur Einstellung der Dosierung von Insulingaben, zugeordnet werden.

7. System nach einem der vorhergehenden Ansprüche, **dadurch gekennzeichnet, dass** die Auswerteeinrichtung (13) ein Handgerät, insbesondere ein PDA, ist, das eine Ausgabeeinheit (17), eine Eingabeeinheit (16) und einen Speicher (15) aufweist.

8. Verfahren zum Auswerten einer Serie von Messwerten der Konzentration eines medizinisch bedeutsamen Analyten in einer menschlichen oder tierischen Körperflüssigkeit, wobei
die Messwerte für Zeitpunkte $t_1$ bis $t_n$, die über einen Zeitraum von mindestens 8 Stunden, vorzugsweise mindestens 24 Stunden, verteilt sind, ermittelt wurden,
aus dem Zeitraum mehrere Zeitintervalle ausgewählt werden, die sich jeweils über mindestens 1 Stunde erstrecken,
aus den Messwerten Messdaten g berechnet werden, wobei als Berechnungsschritt eine lineare Transformation vorgenommen wird, die für unterschiedliche Intervalle individuell festgelegt wird,
**dadurch gekennzeichnet, daß**
für die Zeitintervalle jeweils durch Auswertung von in dem betreffenden Zeitintervall liegenden Messdaten g ein Stabilitätsparameter S ermittelt wird, der die zeitliche Dynamik, mit der sich die Konzentration des Analyten in dem Zeitintervall ändert, charakterisiert, wobei zur Berechnung des Stabilitätsparameters S eines Zeitintervalls in einem Berechnungsschritt für die Zeitintervalle jeweils aus der ersten zeitlichen Ableitung g' oder der zweiten zeitlichen Ableitung g'' der in dem betreffenden Zeitintervall liegenden Messdaten g durch eine Fourier-Transformation ein Powerspektrum berechnet und aus dem Verhältnis der spektralen Intensität hoher Frequenzen zu der spektralen Intensität niedriger Frequenzen der Stabilitätsparamter S berechnet wird, und die Stabilitätsparameter S ausgewertet werden, um krankheitsbedingte Besonderheiten des Stoffwechsels zu ermitteln.

9. Computerprogrammprodukt, das direkt in den Speicher eines digitalen Computers geladen werden kann und Softwareabschnitte umfasst, mit denen die Schritte des Verfahrens nach Anspruch 8 ausgeführt werden, wenn das Produkt auf einem Computer läuft.

**Claims**

1. System for investigation of the metabolism of a human or animal with regard to a medically significant analyte, comprising
a sensor (10) for determining measurement values that are correlated with the concentration of a medically significant analyte in a human or animal body fluid, and
an analytical facility (13) for analyzing a series of measured values provided by the sensor (10),

whereby the analytical facility (13) is adapted such that in operation, it selects from the period of at least 8 hours multiple time intervals, which each extend over at least 1 hour,

and calculates from the measured values measurement data g, wherein a linear transformation is performed as calculation step, said linear transformation being individually set for the various intervals, calculates the first time derivative g' of the measurement data g,

**characterized in that** the analytical facility (13) calculates from the first time derivative g' or from the second time derivative g'' of the measurement data of the respective interval a power spectrum by means of a Fourier transformation and calculates from the relation of spectral intensity of high frequencies to the spectral intensity of low frequencies a stability parameter S that characterizes the time course of the change of the analyte concentration in said time interval, and analyzes the stability parameters S in order to determine disease-related particularities of metabolism.

2. System according to claim 1, **characterized in that** the analytical facility (13) calculates the standard deviation of the values of the derivative over time g' in a further calculation step.

3. System according to any one of the preceding claims, **characterized in that** the analytical facility (13) selects the linear transformation f for each of the individual time intervals such that the mean of the measuring data g of the corresponding time interval corresponds to a pre-determined value.

4. System according to any one of the preceding claims, **characterized in that** the analytical facility (13) selects the linear transformation f for each of the individual time intervals such that the standard deviation of the measuring data g of the corresponding time interval corresponds to a pre-determined value.

5. System according to any one of the preceding claims, **characterized in that** the analyte is glucose.

6. System according to claim 5, **characterized in that** the analytical facility (13) assigns therapeutic recommendations, in particular with regard to the titration of the dosage of insulin doses, to the disease-related particularities of glucose metabolism that have been determined by analysis of the stability parameters.

7. System according to any one of the preceding claims, **characterized in that** the analytical facility (13) is a hand-held device, in particular a PDA, that comprises an output unit (17), an input unit (16), and a memory (15).

8. Method for analyzing a series of measurement values that are correlated with the concentration of a medically significant analyte in a human or animal body fluid, wherein

the measurement values are determined for time points $t_1$ to $t_n$ that are distributed over a period of time of at least 8 hours, preferably at least 24 hours, multiple time intervals each extending over at least 1 hour are selected from the period of time, measurement data g are calculated from the measurement values, wherein a linear transformation is performed as a calculation step, said linear transformation being set individually for various intervals,

**characterized in that**

a stability parameter characterizing the time course of the change of analyte concentration in said time interval is determined for each of the time intervals by analyzing measuring data g that are from said time interval,

wherein for the calculation of the stability parameter S of a time interval in a calculation step for each time interval from the first time derivative g' or from the second time derivative g'' of the measurement data of the respective interval a power spectrum by means of a Fourier transformation is calculated and from the relation of spectral intensity of high frequencies to the spectral intensity of low frequencies the stability parameter S is calculated, and

the stability parameters are analyzed in order to determine disease-related particularities of metabolism.

9. Computer program product that can be loaded directly into the memory of a digital computer and comprises software sections that can be used to perform the steps of the method according to claim 8 when the program runs on a computer.

**Revendications**

1. Système pour l'examen du métabolisme d'un être humain ou d'un animal en ce qui concerne un analyte d'importance médicale, comprenant un capteur (10) pour l'identification de valeurs de mesure de la concentration d'un analyte d'importance médicale dans un fluide corporel humain ou animal, et

un dispositif d'analyse (13) pour l'analyse d'une série de valeurs de mesure identifiées au moyen du capteur (10),

dans lequel le dispositif d'analyse (13) est conçu de telle sorte que, en fonctionnement, il choisisse plusieurs intervalles de temps au sein d'une période de temps d'au moins 8 heures sur laquelle les valeurs de mesure de la série sont réparties, lesquels intervalles de temps s'étendent chacun sur au moins 1 heure, et calcule des données de mesure g à partir des valeurs de mesure, dans lequel une transformation linéaire qui est définie individuellement pour des intervalles différents, qui forme la première dérivée temporelle g' de données de mesure g, est effectuée en tant qu'étape de calcul,

**caractérisé en ce que** le dispositif d'analyse (13) calcule un spectre de puissance pour les intervalles de temps respectivement à partir de la première dérivée temporelle g' ou de la deuxième dérivée temporelle g" des données de mesure g situées dans l'intervalle de temps concerné par l'intermédiaire d'une transformation de Fourier et calcule un paramètre de stabilité S à partir du rapport de l'intensité spectrale de hautes fréquences à l'intensité spectrale de basses fréquences, lequel paramètre de stabilité caractérise la dynamique temporelle avec laquelle la concentration de l'analyte se modifie dans l'intervalle de temps, et

analyse les paramètres de stabilité S afin d'identifier des particularités pathologiques du métabolisme.

2. Système selon la revendication 1, **caractérisé en ce que** la déviation standard des valeurs de la dérivée temporelle g' est calculée par le dispositif d'analyse (13) dans une autre étape de calcul pour la détermination du paramètre de stabilité.

3. Système selon l'une des revendications précédentes, **caractérisé en ce que** la transformation linéaire f est respectivement choisie pour les intervalles de temps individuels par le dispositif d'analyse (13) de telle sorte que la valeur moyenne des données de mesure g de l'intervalle de temps concerné corresponde à une valeur prédéfinie.

4. Système selon l'une des revendications précédentes, **caractérisé en ce que** la transformation linéaire f est respectivement choisie pour les intervalles de temps individuels par le dispositif d'analyse (13) de telle sorte que la déviation standard des données de mesure g de l'intervalle de temps concerné corresponde à une valeur prédéfinie.

5. Système selon l'une des revendications précédentes, **caractérisé en ce que** l'analyte est le glucose.

6. Système selon la revendication 5, **caractérisé en ce que** des recommandations thérapeutiques, en particulier pour l'ajustement de la posologie de doses d'insuline, sont associées par le dispositif d'analyse (13) aux particularités pathologiques du métabolisme du glucose identifiées par analyse des paramètres de stabilité.

7. Système selon l'une des revendications précédentes, **caractérisé en ce que** le dispositif d'analyse (13) est un appareil portatif, en particulier un ordinateur de poche, qui comprend une unité de sortie (17), une unité d'entrée (16) et une mémoire (15).

8. Procédé pour l'analyse d'une série de valeurs de mesure de la concentration d'un analyte d'importance médicale dans un fluide corporel humain ou animal, dans lequel

les valeurs de mesure pour des instants $t_1$ à $t_n$, qui sont réparties sur une période de temps d'au moins 8 heures, de préférence d'au moins 24 heures, ont été identifiées

plusieurs intervalles de temps sont choisis au sein de la période de temps, lesquels s'étendent chacun sur au moins 1 heure,

des données de mesure g sont calculées à partir des valeurs de mesure, dans lequel une transformation linéaire qui est définie individuellement pour des intervalles différents est effectuée en tant qu'étape de calcul, **caractérisé en ce que**

un paramètre de stabilité S est identifié pour les intervalles de temps respectivement par analyse de données de mesure g situées dans l'intervalle de temps concerné, lequel paramètre de stabilité caractérise la dynamique temporelle avec laquelle la concentration de l'analyte se modifie dans l'intervalle de temps, dans lequel, pour le calcul du paramètre de stabilité S d'un intervalle de temps, un spectre de puissance est calculé dans une étape de calcul pour les intervalles de temps respectivement à partir de la première dérivée temporelle g' ou de la deuxième dérivée temporelle g" des données de mesure g situées dans l'intervalle de temps concerné par l'intermédiaire d'une transformation de Fourier et le paramètre de stabilité S est calculé à partir du rapport de l'intensité spectrale de hautes fréquences à l'intensité spectrale de basses fréquences, et

les paramètres de stabilité S sont analysés afin d'identifier des particularités pathologiques du métabolisme.

9. Produit de programme informatique qui peut être chargé directement dans la mémoire d'un ordinateur numérique et comprend des sections de logiciel avec lesquelles les étapes du procédé selon la revendication 8 sont menées, lorsque le produit est exécuté sur un ordinateur.

Fig. 1

Fig. 2

EP 1 793 321 B1

Fig.3

Fig.4

Fig.5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 6272480 B
- EP 1102194 A2
- WO 0215777 A1
- DE 4221848 C2

**In der Beschreibung aufgeführte Nicht-Patentliteratur**

- **B. KOVATCHEV et al.** Quantifying Temporal Glucose Variability in Diabetes via Continuous Glucose Monitoring: Mathematical Methods and Clinical Application. *Diabetes Technology & Therapeutics,* 2005, vol. 7, 849-862
- *Diabetes Technology & Therapeutics,* Dezember 2000, vol. 2 (1), 13-18
- **T. KOSCHINSKY.** Sensors for glucose monitoring: technical and clinical aspects. *Diabetes Metab Res Rev,* 2001, vol. 17, 113-123
- **I. KOHANE.** Hypothesis-Driven Data Abstraction with Trend Templates. *Proc Annu Symp Comput Appl Med Care,* 1993, 444-448
- **G. VELHO et al.** Strategies for calibrating a subcutaneous glucose sensor. *Biomed. Biochim. Acta,* 1989, vol. 48, 957-964
- **A. SIEG et al.** Electroosmosis in Transdermal Iontophoresis: Implications for Noninvasive and Calibration-Free Glucose Monitoring. *Biophysical Journal,* 2004, vol. 87, 3344-3350
- Grundlagen des Diabetes-Managements. **E. STANDL et al.** Diabethologie in Klinik und Praxis. Thieme Verlag, 2003, 132 ff